# EUROPÄISCHE PATENTSCHRIFT

(11) **EP 0 765 473 B1**
(45) Veröffentlichungstag und Bekanntmachung des Hinweises auf die Patenterteilung: **17.12.2003**
(21) Anmeldenummer: 96906743.8
(22) Anmeldetag: 06.03.1996
(51) Int. Cl.: G01N 27/411

(54) **VERFAHREN ZUM MESSEN EINER ELEKTROCHEMISCHEN AKTIVITÄT**
METHOD OF MEASURING ELECTROCHEMICAL ACTIVITY
PROCEDE DE MESURE D'UNE ACTIVITE ELECTROCHIMIQUE

(30) Priorität: 12.04.1995 DE 19513212; 29.08.1995 DE 19531661
(43) Veröffentlichungstag der Anmeldung: 02.04.1997
(73) Patentinhaber: Heraeus Electro-Nite International N.V., 3530 Houthalen (BE)
(72) Erfinder: CURE, Omer, Paul, Ivo, B-3590 Diepenbeek (BE); NEYENS, Guido, Jacobus, B-3680 Maaseik (BE)
(74) Vertreter: Kühn, Hans-Christian
(86) Internationale Anmeldenummer: EP9600952
(87) Internationale Veröffentlichungsnummer: WO96032636

(56) Entgegenhaltungen:
- EP-A- 0 148 492
- EP-A- 0 208 072
- EP-A- 0 450 090
- DE-C- 3 446 320
- RADEX-RUNDSCHAU, Mai 1990, Seiten 236-243, XP002002966 B. KOROUSIC: "Anwendung eines Sauerstoffsensors zur Messung des Sauerstoffpotentials in geschmolzenen ESU-Schlacken" in der Anmeldung erwähnt
- PATENT ABSTRACTS OF JAPAN vol. 8, no. 79 (P-267) [1516] , 11.April 1984 & JP,A,58 223742 (SUMITOMO DENKI KOGYO K.K.), 26.Dezember 1983,

## Beschreibung

Die Erfindung betrifft ein Verfahren zum Messen einer elektrochemischen Aktivität einer auf einer Metallschmelze aufliegenden nichtmetallischen flüssigen Schicht mittels einer Messzelle mit einem elektrochemischen Element, das einen aktiven Teil aufweist und mit einer Gegenelektrode. Die Erfindung betrifft weiterhin eine Messzelle und einen Tauchmessfühler zum Messen einer elektrochemischen Aktivität.

Häufig ist es erforderlich, neben Messungen in Metallschmelzen auch Messungen in oberhalb der Metallschmelze liegenden Schichten durchzuführen. Beispielsweise ist es zur Beurteilung des Ablaufs von metallurgischen Prozessen bei der Stahlherstellung notwendig, das Sauerstoffpotential von Schlacken zu messen. Ebenso kann es erforderlich sein, bei der Aluminiumelektrolyse Aktivitäten in Kryolithschmelzen zu messen, um den metallurgischen Prozess überwachen und steuern zu können.

Aus Radex-Rundschau 1990, Seiten 236 bis 243 ist es bekannt, Sauerstoffmessungen direkt in Schlacke durchzuführen. Dazu wird ein üblicher elektrochemischer Sensor in der Schlacke angeordnet. Der elektrochemische Sensor weist eine Messzelle auf mit einer Gegenelektrode und einem elektrochemischen Element. Das elektrochemische Element ist in bekannter Weise aus einer Ableitelektrode gebildet, die in einem Referenzmaterial angeordnet ist. Dieses Referenzmaterial wiederum ist von einem Festelektrolytröhrchen umgeben. Derartige Messzellen sind beispielsweise auch aus EP 0 108 431 bekannt. Sie dienen gleichermaßen zur Messung von Sauerstoffaktivitäten in Metallschmelzen, wobei die Messzelle selbst während des Durchgangs durch die darüber liegende Schicht (beispielsweise Schlacke) durch eine Schutzkappe geschützt wird.

Die bekannten direkten Messungen von Sauerstoffaktivitäten in über einer Metallschmelze angeordneten geschmolzenen Schichten, wie Schlacke oder Kryolith, erfordern ein exaktes Plazieren des elektrochemischen Elementes innerhalb der zu messenden Schicht. Diese Schicht ist in der Regel relativ dünn (z. B. bei Pfannenschlacke ca. 0 bis 15 cm), so dass abweichende Platzierungen des elektrochemischen Elementes in der Regel auch abweichende Messergebnisse zur Folge haben. Die exakte Anordnung der Messzelle erfordert daher häufig einen relativ großen Aufwand, da auch das Niveau der Oberfläche der Metallschmelze nicht einfach bestimmt werden kann. Die Positionierung erfordert daher eine relativ lange Tauchzeit der Sonde, so dass z. B. die Gegenelektrode und die Messleitungen geschädigt werden können.

Darüber hinaus sind Verfahren zur Analyse von Schlacken bekannt, bei denen Schlackenproben entnommen werden und diese nach dem Erstarren und teilweise nach erneutem Aufschmelzen analysiert werden.

Ausgehend von dem bekannten Stand der Technik liegt der Erfindung die Aufgabe zugrunde ein Verfahren zum Messen einer elektrochemischen Aktivität anzugeben, das mit möglichst geringem Aufwand genaue Messergebnisse liefert. Des weiteren ist es Aufgabe der Erfindung, ein für diese Messungen geeignetes elektrochemisches Element sowie eine Messzelle zur Verfügung zu stellen.

Gelöst wird diese Aufgabe durch die Merkmale der Ansprüche 1, 4 und 13.

Erfindungsgemäß wird die Aufgabe für das eingangs charakterisierte Verfahren dadurch gelöst, dass das elektrochemische Element durch die nichtmetallische, flüssige Schicht hindurch in die Metallschmelze getaucht wird, dass der aktive Teil des elektrochemischen Elements beim Durchgang durch die nichtmetallische flüssige Schicht von dem Material dieser Schicht umgeben wird, dass dieses Material bis nach der Messung der elektrochemischen Aktivität an dem elektrochemischen Element gehalten wird, dass die Messung nach Eintauchen des elektrochemischen Elementes in die Metallschmelze innerhalb der Metallschmelze durchgeführt wird und dass während der Messung mindestens ein Teil der Gegenelektrode direkten Kontakt mit der Metallschmelze hat. Unter einer Messzelle wird eine Anordnung mit mindestens einem elektrochemischen Element und einer Gegenelektrode verstanden, wobei die Gegenelektrode in unmittelbarer Nähe des elektrochemischen Elementes oder davon entfernt angeordnet sein kann. Beispielsweise kann die Gegenelektrode an der Wand des Schmelzbehälters angeordnet oder ein Teil dieser Wand sein. In diesem Fall wird die Gegenelektrode natürlich nicht durch die nichtmetallische flüssige Schicht hindurch in die Schmelze getaucht. Bei diesem Verfahren werden die Messungen in einer annähernd konstanten Umgebung ausgeführt. Eine exakte Platzierung der Messzelle ist nicht erforderlich, da die Metallschmelze in der Regel eine ausreichende Höhe aufweist. Die Temperaturverteilung innerhalb der Metallschmelze ist wesentlich homogener als in der darüberliegenden Schicht, deren Aktivität gemessen werden soll, so dass der Einfluss von Temperaturschwankungen auf das Messergebnis gegenüber dem bekannten direkten Messverfahren vernachlässigbar ist. Die Messungen erfolgen also unter nahezu konstanten Umgebungsbedingungen, so dass reproduzierbare bzw. miteinander vergleichbare Messergebnisse erhalten werden können.

Zur Erhöhung der Genauigkeit und Reproduzierbarkeit von Messungen ist es vorteilhaft, dass während des Durchganges des Eintauchendes des elektrochemischen Elements durch die nicht metallische, flüssige Schicht die Gegenelektrode nicht vollständig mit dem Material dieser Schicht umgeben wird und/oder das dieses Material vor der Messung mindestens teilweise von der Gegenelektrode entfernt wird, so dass die Gegenelektrode direkten Kontakt mit der Metallschmelze hat. Dieses Entfernen kann beispielsweise durch Aufschmelzen erfolgen. Vorteilhaft kann die Sauerstoffaktivität des zu messenden Materials bestimmt werden. Dieses Material der auf einer Metallschmelze aufliegenden nicht metallischen flüssigen Schicht kann beispielsweise bei Eisenschmelzen Schlacke oder bei der Aluminiumelektrolyse Kryolith sein.

Die Aufgabe wird für eine Messzelle zum Messen einer elektrochemischen Aktivität einer auf einer Metallschmelze aufliegenden nichtmetallischen, flüssigen Schicht mit einem an einem Halter angeordneten elektrochemischen Element, das ein Festelektrolytröhrchen mit einem aktiven Teil aufweist, mit einer Gegenelektrode und einer Metallschmelze dadurch gelöst, dass das elektrochemische Element und die Gegenelektrode in der Metallschmelze angeordnet sind, wobei der aktive Teil mit dem Material der zu messenden nicht metallischen Schicht umgeben ist und wobei mindestens ein Teil der Gegenelektrode direkten Kontakt mit der Metallschmelze hat, also nicht mit dem Material der zu messenden nicht metallischen Schicht vollständig umgeben ist. Der direkte Kontakt der Gegenelektrode mit der Metallschmelze bewirkt besonders genaue Messergebnisse. Für exakte Messergebnisse ist es auch vorteilhaft, dass der aktive Teil als ringförmiger Oberflächenbereich des elektrochemischen Elementes ausgebildet ist. Die Gegenelektrode kann vor Gebrauch durch eine Beschichtung, beispielsweise aus Pappe, geschützt sein. Diese Schutzschicht wird beim Durchgang durch die zu messende Schicht oder in der Metallschmelze zerstört und verhindert ein Anhaften des Materials der zu messenden Schicht an der Gegenelektrode.

Zweckmäßig ist es, dass das Eintauchende des Festelektrolytröhrchens mit einem elektrisch isolierenden Material beschichtet ist, wobei ein Bereich zwischen dem Eintauchende und dem feuerfesten Körper beschichtungsfrei ist. Als Beschichtungsmaterialien haben sich beispielsweise Al₂O₃ oder MgO als besonders geeignet erwiesen. Durch eine derartige Beschichtung wird gesichert, dass das Material der zu messenden Schicht an dem elektrochemischen Element haften bleibt, so dass die Aktivitätsmessung in der darunter liegenden Metallschmelze erfolgen kann. Selbstverständlich kann das Material der zu messenden Schicht auch auf andere Weise an dem Festelektrolytröhrchen gehalten werden, beispielsweise durch eine geeignete mechanische Auffangvorrichtung für dieses Material in Form eines das elektrochemische Element beabstandet umgebenden Rohres.

Zweckmäßig ist es, dass das Eintauchende maximal 6 mm in Richtung zum feuerfesten Körper hin beschichtet ist, insbesondere, dass die Beschichtung etwa 2,5 mm lang ist. Vorteilhaft ist es weiterhin, dass das elektrochemische Element aus dem feuerfesten Körper etwa 9 bis 13 mm, insbesondere etwa 11 mm, herausragt. Durch derartige Dimensionierungen wird sowohl ein guter Halt des zu messenden Materials an dem elektrochemischen Element als auch eine zuverlässige Messung der Aktivität dieses Materials gesichert. Zweckmäßig kann es weiterhin sein, dass innerhalb des elektrochemischen Elementes ein Thermoelement angeordnet ist, da die elektrochemische Aktivität auch temperaturabhängig ist und auf diese Weise der Einfluss von evtl. Temperaturschwankungen berücksichtigt werden kann.

Die Aufgabe wird für einen Tauchmessfühler zum Messen einer elektrochemischen Aktivität einer auf einer Metallschmelze aufliegenden, nichtmetallischen, flüssigen Schicht, mit einem an einem Halter angeordneten elektrochemischen Element und einer Gegenelektrode dadurch gelöst, dass an der Gegenelektrode eine Schutzschicht aus einem nicht feuerfesten Material angeordnet ist, so dass während des Eintauchens in die nichtmetallische, flüssige Schicht die Gegenelektrode nicht oder nicht vollständig mit dem Material dieser Schicht umgeben wird und dass das elektrochemische Element beim Eintauchen in die nichtmetallische flüssige Schicht keine Schutzschicht aufweist. Durch die Schutzschicht der Gegenelektrode wird verhindert, dass beim Durchgang durch die Schlackeschicht die Gegenelektrode mit Schlacke bedeckt wird. Die Schutzschicht löst sich in der darunter liegenden Metallschmelze auf, sie verbrennt oder schmilzt beispielsweise. Außer Pappe können andere geeignete Materialien verwendet werden, beispielsweise eine niedrig schmelzende Metallschicht wie Kupfer. Während die Gegenelektrode von der Schlacke nicht bedeckt wird, kann sich die Schlacke an die Oberfläche des elektrochemischen Elementes anlegen und wird mit in die Metallschmelze hineingeführt, da die Schlacke mangels einer Schutzkappe oder ähnlichem auf direktem Wege an das elektrochemische Element gelangt. Es ist möglich, das elektrochemische Element durch eine Schutzschicht oder Schutzkappe, z. B. aus Pappe, gegen mechanische Beschädigung beim Transport usw. zu schützen. Die Schutzkappe wird vor Gebrauch des Tauchmessfühlers abgenommen oder sie verbrennt durch Strahlungswärme vor dem Eintauchen.

Vorteilhaft ist es, dass die Schutzschicht aus Pappe gebildet ist und dass vorzugsweise die gesamte Oberfläche der Gegenelektrode außerhalb des Halters von der Schutzschicht bedeckt ist. Weiterhin ist es zweckmäßig, dass das elektrochemische Element etwa 9 bis 13 mm aus dem Halter herausragt. In einer vorteilhaften Ausgestaltung des Tauchmessfühlers weist der Halter ein Papprohr auf, an dessen Eintauchende ein Messkopf aus einem feuerfesten Material angeordnet ist und es sind das elektrochemische Element und die Gegenelektrode an der Stirnseite des Messkopfes angeordnet, wobei die Gegenelektrode das elektrochemische Element mindestens teilweise ringförmig umgibt, beispielsweise, in dem sie als Metallrohr durch den Messkopf geführt ist und aus diesem an der Stirnseite herausragt. Zweckmäßig ist es weiterhin, dass Kontakte der Gegenelektrode und des elektrochemischen Elementes innerhalb des Halters geführt sind. Diese Kontakte sind in bekannter Weise mit einer Mess- und Auswerteelektronik verbunden.

Die Erfindung bezieht sich auch auf die Verwendung einer Messzelle mit einem in einem Halter angeordneten elektrochemischen Element und mit einer Gegenelektrode zur in einer Metallschmelze erfolgenden Messung der elektrochemischen Aktivität des Materials einer auf der Metallschmelze aufliegenden nichtmetallischen, flüssigen Schicht.

Im folgenden wird ein Ausführungsbeispiel der Erfindung anhand einer Zeichnung näher erläutert. In der Zeichnung zeigt
- Figur 1: eine beispielhafte Ausführung einer erfindungsgemäßen Messzelle mit elektrochemischem Element,
- Figur 2: eine Ausführung mit einem unbeschichteten Elektrolytröhrchen (Durchmesser 5 mm),
- Figur 3: eine Ausführung mit einem unbeschichteten dünnen Elektrolytröhrchen (Durchmesser 3 mm), wobei das Röhrchen nicht über der Gegenelektrode hinausragt,
- Figur 4: eine Ausführung, bei der der aktive Teil als ringförmiger Oberflächenbereich des elektrochemischen Elementes ausgebildet ist,
- Figur 5: ein in einen Schmelzbehälter eingetauchtes Messelement, wobei am elektrochemischen Element Schlacke anhaftet und die Gegenelektrode teilweise abgeschmolzen ist und teilweise durch Schlacke bedeckt wird.

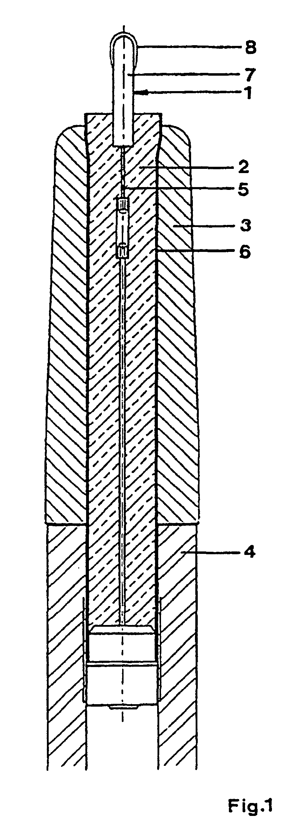

Die Messzelle gemäß Figur 1 weist ein elektrochemisches Element 1 auf, das mittels Zement 2 in einem Halter angeordnet ist. Der Halter ist am Eintauchende der Messzelle aus einem feuerfesten Material 3, beispielsweise aus Gießereisand, und, daran anschließend, aus einem Papprohr 4 gebildet. Aus dem elektrochemischen Element 1 ist eine Elektrode 5 herausgeführt, die mit der Messelektronik, verbunden ist. Die Gegenelektrode 6 ist aus einem Metallrohr gebildet, das die Elektrode 5 umgibt und am Eintauchende der Messzelle aus dem feuerfesten Material 3 des Halters herausragt. Dieser herausragende Teil wird von einer Schicht aus Pappe umgeben, die in der Zeichnung nicht dargestellt ist und die ein Anhaften von Material während des Durchgangs des Eintauchendes der Messzelle durch die auf einer Metallschmelze aufliegende nichtmetallische flüssige Schicht verhindern soll. Das Festelektrolytröhrchen 7 des elektrochemischen Elementes 1 ist an seinem Eintauchende mit einem elektrisch isolierenden Material 8 beschichtet. Diese Schicht besteht im wesentlichen aus MgO, sie kann jedoch auch aus Al₂O₃ oder einem anderen isolierendend Material gebildet sind. Die Schicht ist etwa 50 Mikrometer dick; sie kann auch etwas dicker sein. Die Beschichtung ist in Richtung zum feuerfesten Körper hin etwa 2,5 mm lang. Zwischen dem Bereich, an dem das elektrisch isolierende Material 8 auf dem Festelektrolytröhrchen angeordnet ist und dem feuerfesten Material, in das das Festelektrolytröhrchen 7 eingebettet ist, befindet sich ein etwa 11 mm langer unbeschichteter Bereich, der sogenannte aktive Teil des elektrochemischen Elementes 1.

Zum Messen wird die Messzelle durch die auf einer Stahlschmelze aufliegende Schlackeschicht hindurchgeführt. Dabei wird das elektrochemische Element 1 von der Schlacke umgeben, so dass die Schlacke mit in die Stahlschmelze hineingeführt wird. In der Stahlschmelze stellt sich sehr schnell ein Temperaturgleichgewicht im Bereich des elektrochemischen Elementes 1 ein und die Sauerstoffaktivität der flüssigen Schlackeschicht wird gemessen. Beim Durchgang durch die Schlackeschicht wird durch die an der Gegenelektrode 6 angeordnete Pappe 10 verhindert, dass sich Schlacke an der Gegenelektrode 6 festsetzt. Dadurch steht die Gegenelektrode 6 während der Messung direkt mit der Stahlschmelze in Kontakt.

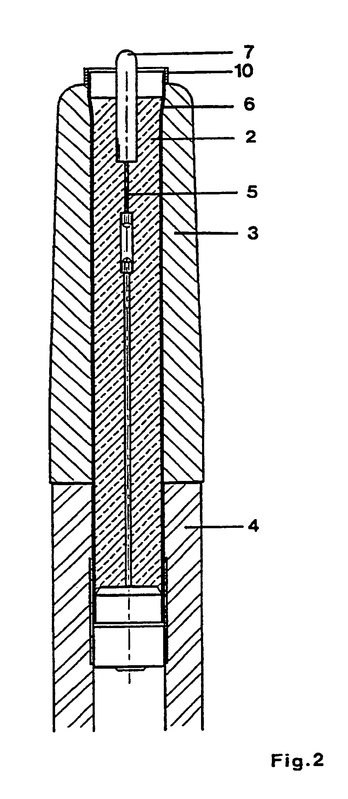

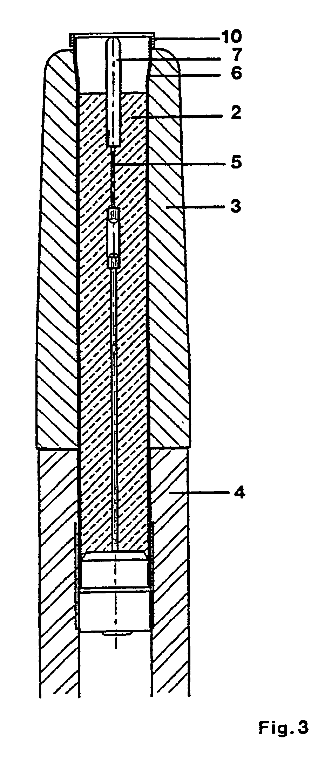

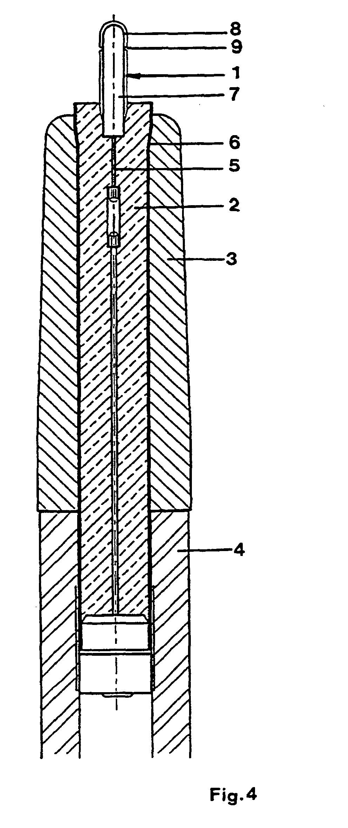

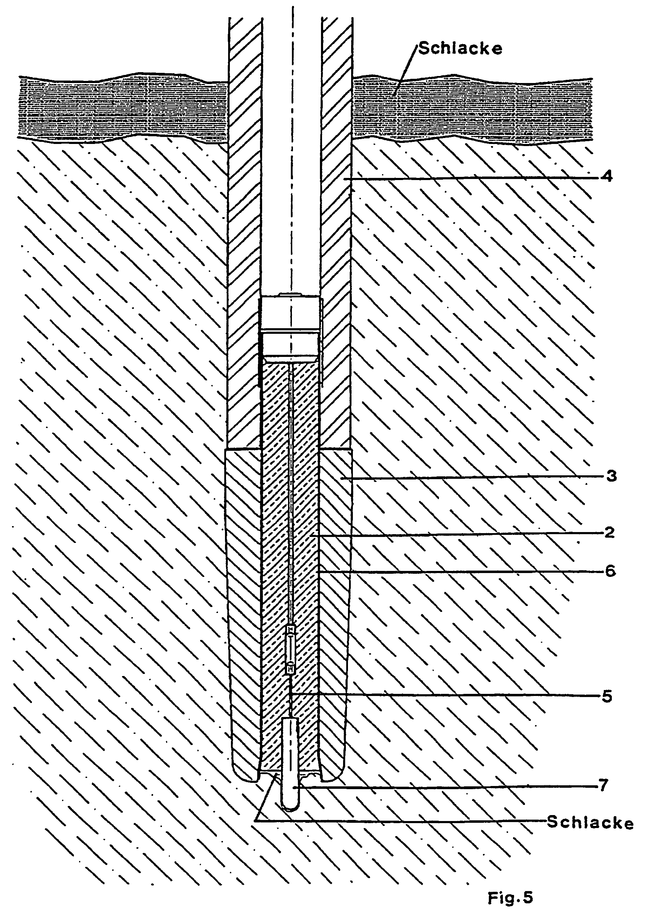

Anstelle des elektrisch isolierenden Materials 8 an der Spitze des elektrochemischen Elementes 1 ist auch die Anwendung anderer Mittel vorstellbar, die ein Anhaften der Schlacke an dem elektrochemischen Element 1 bewirken. Zum Beispiel könnte das elektrochemische Element 1, das etwa 9 - 13 mm, insbesondere 11 mm, aus dem Zement 2 herausragt, unter Bildung eines Ringraumes von einem Röhrchen, zum Beispiel von der durch Pappe 10 geschützten Gegenelektrode 6, umgeben sein, wie in Figur 2 und 3 dargestellt. Dabei muss die Pappe 10 nicht als eine die Gesamtoberfläche der Gegenelektrode 6 umgebende Kappe ausgebildet sein, sondern sie kann auch an der Außenoberfläche des freiliegenden Teils der Gegenelektrode 6, beispielsweise mit Hilfe einer Klebefolie, angeordnet sein. Figur 4 zeigt eine Messzelle, bei der der aktive Teil als ringförmiger Oberflächenbereich 9 des elektrochemischen Elementes ausgebildet ist. In Figur 5 ist die Anordnung des elektrochemischen Elementes in der Metallschmelze dargestellt, wobei am aktiven Teil Schlacke anhaftet, während die Gegenelektrode bereits teilweise abgeschmolzen und ebenfalls teilweise mit Schlacke belegt ist.

## Patentansprüche

1. Verfahren zum Messen einer elektrochemischen Aktivität einer auf einer Metallschmelze aufliegenden nichtmetallischen flüssigen Schicht mittels einer Messzelle mit einem elektrochemischen Element (1), das einen aktiven Teil aufweist und mit einer Gegenelektrode (6), **dadurch gekennzeichnet, dass** das elektrochemische Element durch die nichtmetallische, flüssige Schicht hindurch in die Metallschmelze getaucht wird, damit der aktive Teil des elektrochemischen Elementes (1) beim Durchgang durch die nichtmetallische, flüssige Schicht von dem Material dieser Schicht umgeben wird, dass die Messung nach Eintauchen des elektrochemischen Elementes (1) in die Metallschmelze innerhalb der Metallschmelze durchgeführt wird, dass während der Messung mindestens ein Teil der Gegenelektrode (6) direkten Kontakt mit der Metallschmelze hat, und dass das Material der nichtmetallischen, flüssigen Schicht bis nach der Messung der elektrochemischen Aktivität die Oberfläche des aktiven Teils des elektrochemischen Elementes (1) bedeckt.

2. Verfahren nach Anspruch 1, **dadurch gekennzeichnet, dass** während des Durchgangs des Eintauchendes des elektrochemischen Elements (1) durch die nichtmetallische, flüssige Schicht die Gegenelektrode (6) nicht vollständig mit dem Material dieser Schicht umgeben wird und/oder dass dieses Material vor der Messung mindestens teilweise von der Gegenelektrode (6) entfernt wird.

3. Verfahren nach Anspruch 1 oder 2, **dadurch gekennzeichnet, dass** die Sauerstoffaktivität gemessen wird.

4. Messzelle zum Messen einer elektrochemischen Aktivität einer auf einer Metallschmelze aufliegenden nichtmetallischen, flüssigen Schicht mit einem elektrochemischen Element (1), das an einem mit einem feverfesten Körper versehenen Halter angeordnet ist und ein Festelektrolytröhrchen (7) mit einem aktiven Teil aufweist, mit einer Gegenelektrode (6) und mit einer Metallschmelze, **dadurch gekennzeichnet, dass** das elektrochemische Element (1) und die Gegenelektrode (6) in der Metallschmelze angeordnet sind, wobei der aktive Teil mit dem Material der zu messenden nichtmetallischen Schicht umgeben ist und wobei mindestens ein Teil der Gegenelektrode (6) direkten Kontakt mit der Metallschmelze hat.

5. Messzelle nach Anspruch 4, **dadurch gekennzeichnet, dass** das Eintauchende des Festelektrolytröhrchens (7) mit einem elektrisch isolierenden Material (8) beschichtet ist, wobei ein Bereich zwischen dem Eintauchende und einem Zement (2), mittels dessen das elektrochemische Element (1) in dem Halter angeordnet ist, beschichtungsfrei ist.

6. Messzelle nach Anspruch 5, **dadurch gekennzeichnet, dass** das Eintauchende des Festelektrolytröhrchens (7) mit Al₂O₃ oder MgO beschichtet ist.

7. Messzelle nach Anspruch 5 oder 6, **dadurch gekennzeichnet, dass** das Eintauchende maximal 6 mm in Richtung zum feuerfesten Körper hin beschichtet ist.

8. Messzelle nach Anspruch 7, **dadurch gekennzeichnet, dass** die Beschichtung in Richtung zum feuerfesten Körper hin etwa 2,5 mm lang ist.

9. Messzelle nach einem der Ansprüche 4 bis 8, **dadurch gekennzeichnet, dass** das elektrochemische Element (1) aus dem Zement (2) etwa 9 bis 13 mm herausragt.

10. Messzelle nach Anspruch 9, **dadurch gekennzeichnet, dass** das elektrochemische Element (1) aus dem feuerfesten Körper etwa 11 mm herausragt.

11. Messzelle nach einem der Ansprüche 4 bis 10, **dadurch gekennzeichnet, dass** innerhalb des elektrochemischen Elementes (1) ein Thermoelement angeordnet ist.

12. Messzelle nach Anspruch 4, **dadurch gekennzeichnet, dass** der aktive Teil als ringförmiger Oberflächenbereich (9) des elektrochemischen Elementes (1) ausgebildet ist.

13. Tauchmessfühler zum Messen einer elektrochemischen Aktivität einer auf einer Metallschmelze aufliegenden nichtmetallischen flüssigen Schicht, mit einem an einem Halter angeordneten elektrochemischen Element (1) und einer Gegenelektrode (6), **dadurch gekennzeichnet, dass** an der Gegenelektrode (6) eine Schutzschicht (10) aus einem nicht feuerfesten Material angeordnet ist, so dass während des Eintauchens des Tauchmessfühlers in die nichtmetallische, flüssige Schicht die Gegenelektrode (6) nicht oder nicht vollständig mit dem Material dieser Schicht umgeben wird und ein aktiver Teil des elektrochemischen Elementes (1) keine Schutzschicht aufweist.

14. Tauchmessfühler nach Anspruch 13, **dadurch gekennzeichnet, dass** die Schutzschicht (10) aus Pappe gebildet ist.

15. Tauchmessfühler nach Anspruch 13 oder 14, **dadurch gekennzeichnet, dass** die Schutzschicht (10) die gesamte Oberfläche der Gegenelektrode (6) außerhalb des Halters bedeckt.

16. Tauchmessfühler nach einem der Ansprüche 13 bis 15, **dadurch gekennzeichnet, dass** das elektrochemische Element etwa 9 bis 13 mm aus dem Halter herausragt.

17. Tauchmessfühler nach einem der Ansprüche 13 bis 16, **dadurch gekennzeichnet, dass** der Halter ein Papprohr (4) aufweist, an dessen Eintauchende ein Messkopf aus einem feuerfesten Material (3) angeordnet ist, dass das elektrochemische Element (1) und die Gegenelektrode (6) an der Stirnseite des Messkopfes angeordnet sind und dass die Gegenelektrode (6) das elektrochemische Element (1) mindestens teilweise ringförmig umgibt.

## Claims

1. Method for measuring an electrochemical activity of a nonmetallic liquid layer resting on a metal melt by means of a measuring cell comprising an electrochemical element (1) which has an active part and comprising an opposite electrode (6), **characterized in that** the electrochemical element is immersed through the nonmetallic liquid layer into the metal melt so that the active part of the electrochemical element (1) is surrounded, during passage through the nonmetallic, liquid layer, by the material of this layer, **in that** the measurement is carried out within the metal melt after immersion of the electrochemical element (1) in the metal melt, **in that** at least one part of the opposite electrode (6) is in direct contact with the metal melt during the measurement, and **in that** the material of the nonmetallic, liquid layer covers the surface of the active part of the electrochemical element (1) until after the measurement of the electrochemical activity.

2. Method according to Claim 1, **characterized in that**, during the passage of the immersion end of the electrochemical element (1) through the nonmetallic, liquid layer, the opposite electrode (6) is not completely surrounded by the material of this layer and/or that this material is at least partly removed from the opposite electrode (6) before the measurement.

3. Method according to Claim 1 or 2, **characterized in that** the oxygen activity is measured.

4. Measuring cell for measuring an electrochemical activity of a nonmetallic, liquid layer resting on a metal melt, comprising an electrochemical element (1) which is arranged on a holder provided with a refractory body and a solid electrolyte tube (7) having an active part, comprising an opposite electrode (6) and comprising a metal melt, **characterized in that** the electrochemical element (1) and the opposite electrode (6) are arranged in the metal melt, the active part being surrounded by the material of the nonmetallic layer to be measured and at least a part of the opposite electrode (6) being in direct contact with the metal melt.

5. Measuring cell according to Claim 4, **characterized in that** the immersion end of the solid electrolyte tube (7) is coated with an electrically insulating material (8), a region between the immersion end and a cement (2), by means of which the electrochemical element (1) is arranged in the holder, being free of coatings.

6. Measuring cell according to Claim 5, **characterized in that** the immersion end of the solid electrolyte tube (7) is coated with Al₂O₃ or MgO.

7. Measuring cell according to Claim 5 or 6, **characterized in that** the immersion end is coated not more than 6 mm in the direction of the refractory body.

8. Measuring cell according to Claim 7, **characterized in that** the coating is about 2.5 mm long in the direction of the refractory body.

9. Measuring cell according to any of Claims 4 to 8, **characterized in that** the electrical element (1) projects about 9 to 13 mm from the cement (2).

10. Measuring cell according to Claim 9, **characterized in that** the electrochemical element (1) projects about 11 mm from the refractory body.

11. Measuring cell according to any of Claims 4 to 10, **characterized in that** a thermocouple is arranged inside the electrochemical element (1).

12. Measuring cell according to Claim 4, **characterized in that** the active part is in the form of an annular surface region (9) of the electrochemical element (1).

13. Immersion sensor for measuring an electrochemical activity of a nonmetallic liquid layer resting on a metal melt, comprising an electrochemical element (1) arranged on a holder and an opposite electrode (6), **characterized in that** a protective layer (10) of a nonrefractory material is arranged on the opposite electrode (6) so that, during the immersion of the immersion sensor into the nonmetallic, liquid layer, the opposite electrode (6) is not surrounded, or not completely surrounded, by the material of this layer, and an active part of the electrochemical element(1) has no protective layer.

14. Immersion sensor according to Claim 13, **characterized in that** the protective layer (10) is formed from board.

15. Immersion sensor according to Claim 13 or 14, **characterized in that** the protective layer (10) covers the entire surface of the opposite electrode (6) outside the holder.

16. Immersion sensor according to any of Claims 13 to 15, **characterized in that** the electrochemical element projects about 9 to 13 mm out of the holder.

17. Immersion sensor according to any of Claims 13 to 16, **characterized in that** the holder has a cardboard tube (4) on the immersion end of which a measuring head comprising a refractory material (3) is arranged, that the electrochemical element (1) and the opposite electrode (6) are arranged on the end face of the measuring head and that the opposite electrode (6) surrounds the electrochemical element (1) at least partly in an annular manner.

## Revendications

1. Procédé pour mesurer une activité électrochimique d'une couche liquide non métallique, située sur une masse fondue métallique, à l'aide d'une cellule de mesure comportant un élément électrochimique (1), qui possède une partie active, et une contre-électrode (6), **caractérisé en ce qu'**on immerge l'élément électrochimique à travers la couche liquide non métallique, dans la masse fondue métallique afin que, lors de la traversée de la couche liquide non métallique, la partie active de l'élément électrochimique (1) soit entourée par le matériau de cette couche, qu'on exécute la mesure après immersion de l'élément électrochimique (1) dans la masse fondue métallique, à l'intérieur de la masse fondue métallique, que pendant la mesure, au moins une partie de la contre-électrode (6) est en contact direct avec la masse fondue métallique, et que le matériau de la couche liquide non métallique recouvre, jusqu'après la mesure de l'activité électrochimique, la surface de la partie active de l'élément électrochimique (1).

2. Procédé selon la revendication 1, **caractérisé en ce que** pendant la traversée de la couche liquide non métallique par l'extrémité d'immersion de l'élément électrochimique (1), la contre-électrode (6) n'est pas entourée complètement par le matériau de cette couche et/ou que ce matériau est retiré au moins partiellement de la contre-électrode (6), avant la mesure.

3. Procédé selon la revendication 1 ou 2, **caractérisé en ce qu'**on mesure l'activité de l'oxygène.

4. Cellule de mesure pour mesurer une activité électrochimique d'une couche liquide non métallique, située sur une masse fondue métallique, comportant un élément électrochimique (1), qui est disposé sur un support équipé d'un corps réfractaire et comporte un petit tube à électrolyte solide (7) possédant une partie active, et comportant une contre-électrode (6) et une masse fondue métallique, **caractérisée en ce que** l'élément électrochimique (1) et la contre-électrode (6) sont disposés dans la masse fondue métallique, la partie active étant entourée par le matériau de la couche non métallique à mesurer et au moins une partie de la contre-électrode (6) étant en contact direct avec la masse fondue métallique.

5. Cellule de mesure selon la revendication 4, **caractérisée en ce que** l'extrémité d'immersion du petit tube à électrolyte solide (7) est recouverte avec un matériau électriquement isolant (8), auquel cas une zone située entre l'extrémité d'immersion et un ciment (2), au moyen duquel l'élément électrochimique (1) est disposé dans le support, est exempte de revêtement.

6. Cellule de mesure selon la revendication 5, **caractérisée en ce que** l'extrémité d'immersion du petit tube à électrolyte solide (7) est recouverte de Al₂O₃ ou de MgO.

7. Cellule de mesure selon la revendication 5 ou 6, **caractérisée en ce que** l'extrémité d'immersion est recouverte au maximum sur 6 mm en direction du corps réfractaire.

8. Cellule de mesure selon la revendication 7, **caractérisée en ce que** le revêtement en direction du corps réfractaire possède une longueur d'environ 2,5 mm.

9. Cellule de mesure selon l'une des revendications 4 à 8, **caractérisée en ce que** l'élément électrochimique (1) fait saillie sur environ 9 à 13 mm hors du ciment (2).

10. Cellule de mesure selon la revendication 9, **caractérisée en ce que** l'élément électrochimique (1) fait saillie sur environ 11 mm hors du corps réfractaire.

11. Cellule de mesure selon l'une des revendications 4 à 10, **caractérisée en ce qu'**un thermocouple est disposé à l'intérieur de l'élément électrochimique (12).

12. Cellule de mesure selon la revendication 4, **caractérisée en ce que** la partie active est agencée sous la forme d'une partie de surface annulaire (9) de l'élément électrochimique (1).

13. Capteur de mesure à immersion pour la mesure d'une activité électrochimique d'une couche liquide non métallique située sur une masse fondue métallique, comportant un élément électrochimique (1) disposé sur un support et une contre-électrode (6), **caractérisée en ce qu'**une couche de protection (10) formée d'un matériau non réfractaire est disposée sur la contre-électrode (6), de sorte que pendant l'immersion du capteur de mesure à immersion dans la couche liquide non métallique, la contre-électrode (6) n'est pas entourée ou n'est pas complètement entourée par le matériau de cette couche, et une partie active de l'élément électrochimique (1) ne possède aucune couche de protection.

14. Capteur de mesure à immersion selon la revendication 13, **caractérisé en ce que** la couche de protection (10) est réalisée en carton.

15. Capteur de mesure à immersion selon la revendication 13 ou 14, **caractérisé en ce que** la couche de protection (10) recouvre toute la surface de la contre-électrode (6) à l'extérieur du support.

16. Capteur de mesure à immersion selon l'une des revendications 13 à 15, **caractérisé en ce que** l'élément électrochimique fait saillie sur environ 9 à 13 mm hors du support.

17. Capteur de mesure à immersion selon l'une des revendications 13 à 16, **caractérisé en ce que** le support est un tube en carton (4), sur l'extrémité d'immersion duquel est disposé une tête de mesure formée d'un matériau réfractaire (3), que l'élément électrochimique (1) et la contre-électrode (6) sont disposés sur la face frontale de la tête de mesure et que l'électrode antagoniste (6) entoure au moins partiellement selon une forme annulaire l'élément électrochimique (1).
